# EUROPEAN PATENT SPECIFICATION

(11) **EP 3 011 056 B1**
(45) Date of publication and mention of the grant of the patent: **06.03.2019**
(21) Application number: 14813766.4
(22) Date of filing: 18.06.2014
(51) Int. Cl.: C12Q 1/6834

(54) **REAL-TIME MULTIPLEXED HYDROLYSIS PROBE ASSAY**
ECHTZEIT VERFAHREN UNTER VERWENDUNG VON MULTIPLEX-HYDROLYSE-SONDEN
ANALYSE DE SONDE D'HYDROLYSE MULTIPLEXÉE EN TEMPS RÉEL

(30) Priority: 19.06.2013 US 201361836892 P
(43) Date of publication of application: 27.04.2016
(73) Proprietor: Luminex Corporation, Austin, TX 78727 (US)
(72) Inventor: WHITMAN, Doug, Round Rock, TX 78681 (US); SCHRADER, Brian, Austin, TX 78727 (US)
(74) Representative: D Young & Co LLP
(86) International application number: PCT/US2014/042950
(87) International publication number: WO 2014/205083

(56) References cited:
- WO-A2-2008/074023
- WO-A2-2011/114142
- US-A- 5 210 015
- US-A1- 2008 305 481
- US-A1- 2011 223 602
- US-A1- 2012 088 691
- US-A1- 2013 085 078
- US-A1- 2013 085 078
- WITTWER C T ET AL: "CONTINUOUS FLUORESCENCE MONITORING OF RAPID CYCLE DNA AMPLIFICATION", BIOTECHNIQUES RAPID DISPATCHES, INFORMA HEALTHCARE, US, vol. 22, no. 1, 1 January 1997 (1997-01-01), XP000683698, ISSN: 0736-6205

### SEQUENCE LISTING

<110> LUMINEX CORPORATION
<120> REAL-TIME MULTIPLEXED HYDROLYSIS PROBE ASSAY USING SPECTRALLY IDENTIFIABLE MICROSPHERES
<130> LUMN.P0118WO
<150> US 61/836,892
   <151> 2013-06-19
<160> 9
<170> PatentIn version 3.5
<210> 1
   <211> 18
   <212> DNA
   <213> Artificial sequence
<220>
   <223> Synthetic oligonucleotide
<400> 1
   gtaataatgg cggtggtc 18
<210> 2
   <211> 24
   <212> DNA
   <213> Artificial sequence
<220>
   <223> Synthetic oligonucleotide
<400> 2
   gattgtaaga tttgataaag tgta 24
<210> 3
   <211> 27
   <212> DNA
   <213> Artificial sequence
<220>
   <223> Synthetic oligonucleotide
<400> 3
   gaccaccgcc attattacga acagctg 27
<210> 4
   <211> 24
   <212> DNA
   <213> Artificial sequence
<220>
   <223> Synthetic oligonucleotide
<400> 4
   tacactttat caaatcttac aatc 24
<210> 5
   <211> 21
   <212> DNA
   <213> Artificial sequence
<220>
   <223> Synthetic oligonucleotide
<400> 5
   tcagcagttg aagggacaga t 21
<210> 6
   <211> 21
   <212> DNA
   <213> Artificial sequence
<220>
   <223> Synthetic oligonucleotide
<400> 6
   ccagaacaat gaatggttaa g 21
<210> 7
   <211> 19
   <212> DNA
   <213> Artificial sequence
<220>
   <223> Synthetic oligonucleotide
<400> 7
   gaccaccgcc attattacg 19
<210> 8
   <211> 27
   <212> DNA
   <213> Artificial sequence
<220>
   <223> Synthetic oligonucleotide
<400> 8
   cagctgttcg taataatggc ggtggtc 27
<210> 9
   <211> 180
   <212> DNA
   <213> S. epidermidis
<400> 9
   atctactact tcaccttttt cttcagaatt tggtgataat agttcccaga acaatgaatg        60
   gttaaggtga ccaccgccat tattacgaac agctgtttga atattagatg gcacactatc       120
   taaattagca acaatttctt cgattgattt agcttctaaa tctgtccctt caactgctga       180

## Description

### BACKGROUND OF THE INVENTION

### 1. Field of the Invention

The present invention relates generally to the field of molecular biology. More particularly, it concerns the detection and quantification of nucleic acids.

### 2. Description of Related Art

Polymerase chain reaction (PCR) is a molecular biology technique commonly used in medical and biological research labs for a variety of tasks, such as the detection of hereditary diseases, the identification of genetic fingerprints, the diagnosis of infectious diseases, the cloning of genes, paternity testing, and DNA computing. PCR has been accepted by molecular biologists as the method of choice for nucleic acid detection because of its unparalleled amplification and precision capability. DNA detection is typically performed at the end-point, or plateau phase of the PCR reaction, making it difficult to quantify the starting template. Real-time PCR or kinetic PCR advances the capability of end-point PCR analysis by recording the amplicon concentration as the reaction progresses. Amplicon concentration is most often recorded via a fluorescent signal change associated with the amplified target. Real-time PCR is also advantageous over end-point detection in that contamination is limited because it can be performed in a closed system. Other advantages include greater sensitivity, dynamic range, speed, and fewer processes required.

Several assay chemistries have been used in real-time PCR detection methods. These assay chemistries include using double-stranded DNA binding dyes, dual-labeled oligonucleotides, such as hairpin primers, and hairpin probes. However, a drawback of many real-time PCR technologies is limited multiplexing capability. Real-time PCR technologies that use reporter fluorochromes that are free in solution require a spectrally distinct fluorochrome for each assay within a multiplex reaction. For example, a multiplex reaction designed to detect 4 target sequences would require an instrument capable of distinguishing 4 different free floating fluorochromes by spectral differentiation, not including controls. These requirements not only limit the practical multiplexing capability, but also increase costs since such instruments typically require multiple lasers and filters.

US 2013/085078 A1 discloses methods and compositions for the detection and quantification of nucleic acids. In one embodiment, a sample is contacted with a primer complementary to a first region of a target nucleic acid and a probe complementary to a second region of the target nucleic acid downstream of the first region under conditions suitable for hybridization of the target nucleic acid with the primer and the probe. The probe in this embodiment comprises a fluorophore and is attached to a solid support. The hybridized probe is cleaved with a nucleic acid polymerase having exonuclease activity to release the reporter from the solid support. The presence of the target nucleic acid is then detected and optionally quantified by detecting a decrease in signal from the reporter on the solid support.

In Wittwer et al., 1997. "Continuous fluorescence monitoring of rapid cycle DNA amplification". Biotechniques, 22(1), pp. 130-139 rapid cycle DNA amplification was continuously monitored by three different fluorescence techniques. Fluorescence was monitored by (i) the double-strand-specific dye SYBR Green I, (ii) a decrease in fluorescein quenching by rhodamine after exonuclease cleavage of a dual-labeled hydrolysis probe and (iii) resonance energy transfer of fluorescein to Cy5 by adjacent hybridization probes.

### SUMMARY OF THE INVENTION

The invention is defined by the appended claims. In certain embodiments, methods of detecting nucleic acids are provided. In a first embodiment, a method is provided for detecting a target nucleic acid in a sample, comprising: (a) contacting the sample with a first target-specific primer complementary to a first region on a first strand of the target nucleic acid, a target-specific probe complementary to a second region on the first strand of the target nucleic acid downstream of the first region, and a second target-specific primer complementary to a region on a second strand of the target nucleic acid under conditions suitable for hybridization of the target nucleic acid with the first target-specific primer, the target-specific probe and the second target-specific primer, wherein the target-specific probe comprises a quencher and wherein the 3' end of the target-specific probe comprises a modification to prevent polymerase extension of the probe; (b) performing a polymerase chain reaction (PCR) with a nucleic acid polymerase having exonuclease activity; (c) cleaving the hybridized target-specific probe with said nucleic acid polymerase to release the quencher from the target-specific probe; (d) hybridizing any remaining target-specific probe to a reporter probe that is complementary to the target-specific probe, said reporter probe comprising a reporter and being attached to a solid support, wherein the reporter is a fluorophore; and (e) detecting the target nucleic acid by detecting a change in signal from the reporter in association with the solid support (*e*.*g*., a bead); wherein the hybridization temperature of the target-specific probe for the target nucleic acid molecule is higher than the hybridization temperature of the target-specific probe for the reporter probe.

In one aspect, the first target-specific primer and the target-specific probe hybridize to adjacent sequences on the target nucleic acid. In another aspect, the first target-specific primer and the target-specific probe hybridize to non-adjacent sequences on the target nucleic acid. For example, in the later case a method can further comprise extending the first target-specific primer with the nucleic acid polymerase having exonuclease activity.

In one aspect, the solid support may be a bead, such as an encoded bead. In some aspects the solid support is attached the 3' end of a reporter probe. For example, the fluorophore may be attached at the 5' end of the reporter probe. When the reporter is a fluorophore, the change in the signal may be an increase in the fluorescent signal. In some aspects, a reporter probe comprises a linker between the reporter probe and the solid support.

In further aspects, the target-specific probe and the reporter probe may have a known hybridization temperature. For example, in some aspects, the hybridization temperature of the reporter probe to the target specific probe is lower than the annealing temperature in the PCR, and the hybridization temperature of the reporter probe to the target nucleic acid is below the annealing temperature in the PCR. In some aspects, detecting a change in signal from the reporter may comprise detecting the rate of change in signal from the reporter as the temperature of the sample is changed. For instance, detecting a change in signal from the reporter may comprise detecting the rate of change in signal from the reporter as the temperature of the sample is increased above (or decreased below) the hybridization temperature of the target-specific probe and the reporter probe. In some aspects, detecting a change in signal comprises detecting a shift in the temperature at which a hybridization or melt peak is observed. In still further aspects, a method comprises determining the area under the curve of the hybridization or melt peak of either or both of the cleaved and/or uncleaved target-specific probe. Thus, in some aspects, determining the presence or absence of the target nucleic acid comprises comparing a ratio of the area under the curve of the hybridization or melt peaks of the cleaved and uncleaved target-specific probe.

In certain aspects, a method further comprises detecting a reference signal from a distinct reporter on a non-hybridizing probe attached to a solid support. In one aspect, the non-hybridizing probe may be attached to a spatially discrete location on the same solid support to which the second probe is attached. In another aspect, the non-hybridizing probe may be attached to a different solid support than that to which the reporter probe is attached. In this aspect, the different solid supports may be different encoded beads. In certain aspects, the method further comprises using the reference signal to normalize for changes in fluorescence over time.

In further aspects, a method further comprises detecting a signal from the reporter probe on the solid support prior to cleaving the hybridized target-specific probe.

In one embodiment a method may be performed to detect a single target. Alternatively, additional primers and/or probes may be included to detect multiple distinct target nucleic acids in a multiplex assay. For example, in one aspect, the target nucleic acid is a first target nucleic acid, the quencher is a first quencher, the reporter probe is a first reporter probe, the reporter is a first reporter, the solid support is a first solid support, and the method further comprises: (a) contacting the sample with a third target-specific primer complementary to a first region on a first strand of a second target nucleic acid, a second target-specific probe complementary to a second region on the first strand of the second target nucleic acid downstream of the first region, and a fourth target-specific primer complementary to a region on a second strand of the second target nucleic acid under conditions suitable for hybridization of the second target nucleic acid with the third target-specific primer, the second target-specific probe, and the fourth target-specific primer wherein the second target-specific probe comprises a second quencher; (b) performing a polymerase chain reaction (PCR) with a nucleic acid polymerase having exonuclease activity; (c) cleaving the second hybridized target-specific probe with said nucleic acid polymerase to release the second quencher from the second target-specific probe; (d) hybridizing any remaining second target-specific probe to a second reporter probe that is complementary to the second target-specific probe, said second reporter probe comprising a second reporter and being attached to a second solid support, wherein the second reporter is a fluorophore; and (e) detecting the second target nucleic acid by detecting a chance in signal from the second reporter associated with the second solid support.

In some aspects, the first solid support and the second solid support may be spatially discrete locations on one solid support, such as spatially discrete locations on a planar array. In another aspect, the first solid support may be physically separate from the second solid support, such as with a bead array. In some aspects, the reporters attached to the different reporter probes may be the same because the different reporter probes can be distinguished by the solid support(s) to which they are attached. In some aspects, however, two or more different reporters are used.

In still further aspects, different reporter probes having the same reporter can be distinguished from one another by having different melting temperatures with their corresponding target-specific probes. In these aspects, the first target-specific probe and the first reporter probe have a first hybridization temperature wherein the second target-specific probe and the second reporter probe have a second hybridization temperature and wherein said first and second hybridization temperatures are different. The first and second hybridization temperatures may be separated by at least about 3, 5, 7, or 10 degrees. In one aspect, detecting a change in signal from the first or second reporter may comprise detecting the rate of change in signal from the reporter as the temperature of the sample is changed. For example, detecting a change in signal from the first or second reporter may comprise detecting the rate of change in signal from the first and/or second reporter as the temperature of the sample is increased above (or decreased below) the first and/or second hybridization temperature.

As described herein there is provided a method for detecting a target nucleic acid in a sample, comprising: (a) contacting the sample with a first target-specific primer complementary to a first region on a first strand of the target nucleic acid, and a target-specific probe complementary to a second region on the first strand of the target nucleic acid downstream of the first region under conditions suitable for hybridization of the target nucleic acid with the first target-specific primer and the target-specific probe, wherein the target-specific probe comprises a tag at its 5' or 3' end and a quencher; (b) cleaving the hybridized target-specific probe with a nucleic acid polymerase having exonuclease activity to release the quencher from the tag; (c) hybridizing the tag to a complementary anti-tag immobilized on a solid support and comprising a reporter; and (d) detecting the target nucleic acid by detecting an increase in signal from the reporter on the solid support. As described supra, the first target-specific primer and the target-specific probe can hybridize to adjacent or non-adjacent sequences on the target nucleic acid. In the case, of non-adjacent hybridization a method further comprises extending the first target-specific primer with the nucleic acid polymerase having exonuclease activity.

The method may further comprise hybridizing the target-specific probe to the anti-tag immobilized on the solid support prior to cleaving the hybridized target-specific probe to release the quencher molecule from the tag; and detecting a signal from the reporter on the solid support. The reporter may be a biotin or other ligand bound to a fluorophore or a directly coupled fluorophore. In one aspect, the solid support may be a bead. A reporter probe may further comprise a linker between the probe and the solid support.

The tag may be a nucleic sequence and the anti-tag may be a nucleic acid sequence complementary to the tag sequence. Thus, the tag and the anti-tag may have a known hybridization temperature and detecting a change in signal from the reporter may comprise detecting the rate of change in signal from the reporter as the temperature of the sample is changed. For instance, detecting a change in signal from the reporter may comprise detecting the rate of change in signal from the reporter as the temperature of the sample is increased above (or decreased below) the hybridization temperature of the tag and the anti-tag.

In some aspects, a method of the embodiments further comprises contacting the sample with a second target-specific primer complementary to a region on a second strand of the target nucleic acid. The first target-specific primer and the second target-specific primer are oriented on opposite strands of the target nucleic acid such that the region of the target nucleic acid can be amplified by PCR. In a further aspect, the method comprises performing multiple polymerase chain reaction cycles. In a related aspect, a method comprises detecting a signal (or change in signal) two or more times over multiple polymerase chain reaction cycles (*e*.*g*., detecting a signal or change in signal continuously over the PCR cycles). A typical amplification cycle has three phases: a denaturing phase, a primer annealing phase, and a primer extension phase, with each phase being carried out at a different temperature. In some aspects, a 2-stage PCR also may be performed in which only two temperatures are used for each cycle; e.g., 95°C and 60°C. Thus, in certain aspects the method further comprises repeatedly hybridizing the target nucleic acid with the target-specific primers and the target-specific probe, extending the target-specific primers with the nucleic acid polymerase having exonuclease activity such that extension of the first target-specific primer results in the cleavage of the hybridized target-specific probe and release the quencher from the target-specific probe, and detecting the change in signal from the reporter probe on the solid support. In certain embodiments amplification cycles are repeated at least until the change in the signal is distinguishable from background noise. Although, if a particular target nucleic acid is not present in the sample, then the change in signal should not be distinguishable from background noise regardless of the number of cycles performed. The inclusion of appropriate positive and negative controls in the reaction can assist in determining that a particular target nucleic acid is not present in the sample. A person of ordinary skill in the art will know how to select the appropriate positive and negative controls for a particular assay.

In further aspects, the multiple polymerase chain reaction cycles can be performed without a wash step to remove free-floating quencher between cycles (*e*.*g*., in closed container, such as a tube). In some aspects, detecting the change in signal from the reporter on the solid support comprises detecting the signal before and after performing the multiple polymerase chain reaction cycles. In another aspect, detecting the change in signal from the reporter on the solid support comprises detecting the signal only after performing the multiple polymerase chain reaction cycles. In this aspect, the method may further comprise comparing the detected signal from the reporter on the solid support to a predetermined ratio of the signal of the reporter on the solid support to a reference signal from a reporter on a non-hybridizing probe attached to a solid support. Thus, in some aspects, a method comprises detecting a signal (or change in signal) two or more times over multiple polymerase chain reaction cycles (*e*.*g*., detecting a signal or change in signal continuously over the PCR cycles).

A method comprising multiple polymerase chain reaction cycles may, in some cases, further comprise quantifying the amount of the target nucleic acid in the sample. In one aspect, quantifying the amount of the target nucleic acid in the sample comprises using a standard curve. In another aspect, quantifying the amount of the target nucleic acid in the sample comprises determining a relative amount of the target nucleic acid. In yet another aspect, quantifying the amount of the target nucleic acid in the sample comprises using end-point detection of the presence or absence of a target nucleic acid by relating the change in signal from the reporter on the solid support to a reference signal from a reporter on a non-hybridizing probe attached to a solid support. In particular embodiment, the detected signal from the reporter on the solid support is compared to a predetermined ratio of the signal of the reporter on the solid support to a reference signal from a reporter on a non-hybridizing probe attached to a solid support. Determining that the ratio has changed would indicate the presence of the target nucleic acid in the assay. An advantage of this approach is that it can be performed without requiring multiple images (*e*.*g*., one image before amplification and one image after amplification). In certain aspects, the predetermined ratio is stored in a computer-readable medium and accessed by software analyzing data relating to the signals from the reporter molecules. A "non-hybridizing probe" is a probe that has a sequence that is not expected to hybridize to any other nucleic acids present in the assay under assay conditions.

In yet further aspects, quantifying the amount of the target nucleic acid in the sample comprises determining an amount of the target nucleic acid by relating the PCR cycle number at which the signal is detectable over background to the amount of target present. This method may be performed to detect a single target or additional primers and probes may be included to detect multiple different target nucleic acids in a multiplex assay. For example, as described herein, the target nucleic acid is a first target nucleic acid, the quencher is a first quencher, the reporter is a first reporter, the tag is a first tag, the anti-tag is a first anti-tag, the solid support is a first solid support, and the method further comprises: (a) contacting the sample with a second target-specific primer complementary to a first region on a first strand of a second target nucleic acid, and a second target-specific probe complementary to a second region on the first strand of the second target nucleic acid downstream of the first region under conditions suitable for hybridization of the second target nucleic acid with the second target-specific primer and the second target-specific probe, wherein the second target-specific probe comprises a second tag at its 5' or 3' end and a second quencher; (b) cleaving the second hybridized target-specific probe with the nucleic acid polymerase having exonuclease activity to release the second quencher from the second tag; (c) hybridizing the second tag to a complementary second anti-tag immobilized on a second solid support and comprising a second reporter; and (d) detecting the second target nucleic acid by detecting an increase in signal from the second reporter on the second solid support.

As described herein there is provided a method for quantifying an amount of a target nucleic acid in a sample, comprising: (a) amplifying the target nucleic acid in the presence of a nucleic acid polymerase having exonuclease activity, a target-specific primer pair comprising a first primer complementary to a first region on a first strand of the target nucleic acid and a second primer complementary to a region on a second strand of the target nucleic acid, and a target-specific probe complementary to a second region on the first strand of the target nucleic acid downstream of the first region, wherein the target-specific probe comprises a quencher, and further wherein the nucleic acid polymerase cleaves the target-specific probe and releases the quencher from the target-specific probe when extending the first primer along the first strand of the target nucleic acid; (b) hybridizing the remaining target-specific probe to a reporter probe that is complementary to the target-specific probe, said reporter probe comprising a reporter and being attached to a solid support; (c) detecting a first signal from the reporter on the solid support at a first time and a second signal from the reporter on the solid support at a second time; (d) correlating a change in signal with the amount of the target nucleic acid in the sample. The method may comprise quantifying an amount of a plurality of different target nucleic acids in the sample. The method may comprise quantifying the amount of the target nucleic acid in the sample using a standard curve or determining a relative amount of the target nucleic acid. The method may further comprise detecting at least a third signal from the reporter on the solid support at a third time.

In further aspects, a method comprises detecting a signal from the reporter on the solid support prior to extending the target-specific primer with the nucleic acid polymerase having exonuclease activity to cleave the hybridized target-specific probe and release the quencher from the target-specific probe. In some aspects, the target-specific probe and the reporter probe have a known hybridization temperature and wherein detecting a first or second signal from the reporter comprises detecting a first or second rate of change in signal from the reporter as the temperature of the sample is changed. For instance, detecting a first or second signal from the reporter comprises detecting the first or second rate of change in signal from the reporter as the temperature of the sample is increased above the hybridization temperature of the target-specific probe and the reporter probe.

As described herein a method is provided for quantifying an amount of a target nucleic acid in a sample, comprising: (a) amplifying the target nucleic acid in the presence of a nucleic acid polymerase having exonuclease activity, a target-specific primer pair comprising a first primer complementary to a first region on a first strand of the target nucleic acid and a second primer complementary to a region on a second strand of the target nucleic acid, and a target-specific probe complementary to a second region on the first strand of the target nucleic acid downstream of the first region under conditions suitable for hybridization of the target nucleic acid with the target-specific primer and the target-specific probe, wherein the target-specific probe comprises a tag at its 5' or 3' end and a quencher, and further wherein the nucleic acid polymerase cleaves the target-specific probe and releases the quencher from the target-specific probe when extending the first primer along the first strand of the target nucleic acid; (b) hybridizing the tag to a complementary anti-tag immobilized on a solid support and comprising a reporter; (c) detecting a first signal from the reporter on the solid support at a first time and a second signal from the reporter on the solid support at a second time; (d) correlating a change in signal with the amount of the target nucleic acid in the sample. The method may comprise quantifying an amount of a plurality of different target nucleic acids in the sample.

In some aspects, quantifying the amount of the target nucleic acid in the sample comprises using a standard curve or determining a relative amount of the target nucleic acid. In certain aspects, a method further comprises detecting at least a third signal from the reporter on the solid support at a third time.

In some aspects, the method comprises detecting a signal from the reporter on the solid support prior to extending the target-specific primer with the nucleic acid polymerase having exonuclease activity to cleave the hybridized target-specific probe and release the quencher from the target-specific probe.

As described herein there is provided a composition comprising at least two different primer-probe sets, wherein each primer-probe set comprises: (i) a first primer complementary to a first region on a first strand of a target nucleic acid; (ii) a second primer complementary to a region on a second strand of the target nucleic acid; (iii) a labeled target-specific probe, wherein the labeled target-specific probe is capable of specifically hybridizing to a second region on the first strand of the target nucleic acid, wherein the second region is downstream of the first region; and (iv) a labeled anti-probe covalently attached to a particle, wherein the labeled anti-probe is capable of specifically hybridizing to the labeled target-specific probe.

In some aspects, the labeled target-specific probe quenches the signal from the labeled anti-probe when the target-specific probe and anti-probe are hybridized. In one aspect, the particle is a distinguishably encoded particle. In one aspect, the composition further comprises a polymerase with 5' exonuclease activity. In another aspect, the composition further comprises a passive reference probe covalently attached to the particle. In yet another aspect, the composition comprises at least four different primer-probe sets.

As described herein there is provided a kit comprising at least two different primer-probe sets, wherein each primer-probe set comprises: (i) a first primer complementary to a first region on a first strand of a target nucleic acid; (ii) a second primer complementary to a region on a second strand of the target nucleic acid; (iii) a labeled target-specific probe, wherein the labeled target-specific probe is capable of specifically hybridizing to a second region on the first strand of the target nucleic acid, wherein the second region is downstream of the first region; and (iv) a labeled anti-probe covalently attached to a particle, wherein the labeled anti-probe is capable of specifically hybridizing to the labeled target-specific probe.

In some aspects, the labeled target-specific probe quenches the signal from the labeled anti-probe when the target-specific probe and anti-probe are hybridized. In another aspect, the labeled anti-probes of the two different primer-probe sets comprise labels that are distinguishable from one another. In certain aspects, the two different primer-probe sets comprise have different probe-anti-probe hybridization temperatures (e.g., hybridization temperatures differing by at least 2, 3, 4, 5, 6, 7, 8, 9, 10 or more degrees). In some aspects, the kit further comprises a polymerase with exonuclease activity. In one aspect, the kit further comprises a passive reference probe covalently attached to a distinguishably encoded particle. In one aspect, the kit further comprises at least eight different primer-probe sets.

As described herein there is provided a multiplex method for detecting the presence or absence of a plurality of target nucleic acids in a sample, comprising: (a) contacting the sample with a plurality of primer/probe pairs, each primer/probe pair comprising a target-specific primer complementary to a first region on a first strand of one of the plurality of target nucleic acids, and a target-specific probe complementary to a second region on the first strand of one of the plurality of target nucleic acids downstream of the first region under conditions suitable for hybridization of the target nucleic acid with the first target-specific primer and the target-specific probe, wherein the target-specific probe comprises a quencher; (b) cleaving the hybridized target-specific probes with a nucleic acid polymerase having exonuclease activity to release the quenchers from the target-specific probes; (c) hybridizing the remaining target-specific probes to reporter probes that are complementary to the target-specific probes, said reporter probes comprising reporters and being attached to solid supports; and (d) detecting signals from the reporters on the solid support, whereby an increase in the signal indicates the presence of a target nucleic acid.

In one aspect, the solid support is a bead, such as an encoded bead. In one aspect, the reporter is a fluorophore. In a further aspect, each reporter probe comprises the same fluorophore. In some aspects, two or more of the reporter probes comprise the same fluorophore and said two or more reporter probes have different hybridization temperatures with their corresponding target-specific probe. In one aspect, the change in the signal is an increase in a fluorescent signal.

In one aspect, each pair of target-specific probes and reporter probes have a known hybridization temperature and detecting signals from the reporters comprises detecting the rate of change in signals from the reporters as the temperature of the sample is changed. In a further aspect, detecting signals from the reporters comprises detecting the rate of change in signals from the reporters as the temperature of the sample is increased above (or decreased below) the hybridization temperature of each pair of target-specific probes and reporter probes.

In some aspects, each different target-specific probe of the plurality of primer/probe pairs is attached to a spatially discrete location on one solid support. In certain aspects, each different target-specific probe of the plurality of primer/probe pairs is attached to a different solid support. In one aspect, the method further comprises detecting a reference signal from a reporter on a non-hybridizing probe attached to a solid support. In a further aspect, the non-hybridizing probe is attached to a spatially discrete location on the same solid support to which the target-specific probes are attached. In another aspect, the non-hybridizing probe is attached to a different solid support than that to which the target-specific probes are attached.

In another aspect, each different target-specific probe of the plurality of primer/probe pairs comprises the same quencher. In another aspect, each different target-specific probe of the plurality of primer/probe pairs comprises a different quencher. In one aspect, the method further comprises contacting the sample with a plurality of different second target-specific primers complementary to a region on a second strand of the plurality of target nucleic acids, and performing multiple polymerase chain reaction cycles. In some cases, the multiple polymerase chain reaction cycles are performed without a wash step to remove free-floating quenchers between cycles (*e*.*g*., in closed container, such as a tube). In certain aspects, detecting the changes in signal from the reporters on the solid support comprises detecting the signals before and after performing the multiple polymerase chain reaction cycles. In another aspect, detecting the changes in signals from the reporters on the solid support comprises detecting the signals only after performing the multiple polymerase chain reaction cycles. In certain aspects, a method comprises detecting a signal (or change in signal) two or more times over multiple polymerase chain reaction cycles (*e*.*g*., detecting a signal or change in signal continuously over the PCR cycles). In some aspects, the method further comprises comparing the detected signals from the reporters on the solid support to a predetermined ratio of the signal of the reporter on the solid support to a reference signal from a reporter on a non-hybridizing probe attached to a solid support. In one aspect, the method further comprises quantifying the amount of the target nucleic acid in the sample.

In certain aspects, one or more controls are included in the reaction. For example, in some embodiments, a method for detecting a target nucleic acid in a sample may further comprise detecting a signal from a reporter on a non-hybridizing (*i.e.,* negative control) probe attached to a solid support. The non-hybridizing probe may be attached to a spatially discrete location on the same solid support to which the target-specific probe is attached, attached to a different solid support than that to which the target-specific probe is attached, or otherwise distinguishable from the target-specific probe. In certain aspects, the different solid supports are different encoded beads.

The target nucleic acid may be any sequence of interest. In some aspects, the nucleic acid is a DNA. In some aspects, the nucleic acid is an RNA. The sample containing the target nucleic acid may be any sample that contains nucleic acids. In certain aspects of the invention the sample is, for example, from a subject who is being screened for the presence or absence of one or more genetic mutations or polymorphisms. In another aspect of the invention the sample may be from a subject who is being tested for the presence or absence of a pathogen. Where the sample is obtained from a subject, it may be obtained by methods known to those in the art such as aspiration, biopsy, swabbing, venipuncture, spinal tap, fecal sample, or urine sample. In some aspects of the invention, the sample is an environmental sample such as a water, soil, or air sample. In other aspects of the invention, the sample is from a plant, bacterium, virus, fungus, protozoan, or metazoan. The term target nucleic acid encompasses both an unamplified sequence and amplicons thereof.

A quencher as used herein is a moiety that absorbs and thereby decreases the apparent intensity of a fluorescence moiety when in close proximity to a fluorescence moiety. In some aspects, a quencher for use according to the embodiments emits the absorbed fluorescence in different spectrum. Thus, in some aspects, a detection method of the embodiments employs a filter that to reduce or remove fluorescence emitted by a quencher. In certain aspects, a quencher is a dark quencher with no native fluorescence and therefore do not occupy an emission bandwidth. Such a dark quencher is a substance that absorbs excitation energy from a fluorophore and dissipates the energy as heat. Examples of dark quenchers include, but are not limited to, Dabsyl, Black Hole Quenchers, Qxl quenchers, Iowa black FQ, Iowa black RQ, and IRDye QC-1.

A reporter, which may also be referred to as a labeling agent, is a molecule that facilitates the detection of another molecule (*e.g.,* a nucleic acid) to which it is attached. Numerous reporter molecules that may be used to label nucleic acids are known. Direct reporter molecules include fluorophores, chromophores, and radiophores. Non-limiting examples of fluorophores include, a red fluorescent squarine dye such as 2,4-Bis[1,3,3-trimethyl-2-indolinylidenemethyl] cyclobutenediylium-1,3-dioxolate, an infrared dye such as 2,4 Bis [3,3-dimethyl-2-(1H-benz[e]indolinylidenemethyl)] cyclobutenediylium-1,3-dioxolate, or an orange fluorescent squarine dye such as 2,4-Bis [3,5-dimethyl-2-pyrrolyl] cyclobutenediylium-1,3-diololate. Additional non-limiting examples of fluorophores include quantum dots, Alexa Fluor® dyes, AMCA, BODIPY® 630/650, BODIPY® 650/665, BODIPY®-FL, BODIPY®-R6G, BODIPY®-TMR, BODIPY®-TRX, Cascade Blue®, CyDye™, including but not limited to Cy2™, Cy3™, and Cy5™, a DNA intercalating dye, 6-FAM™, Fluorescein, HEX™, 6-JOE, Oregon Green® 488, Oregon Green® 500, Oregon Green® 514, Pacific Blue™, REG, phycobilliproteins including, but not limited to, phycoerythrin and allophycocyanin, Rhodamine Green™, Rhodamine Red™, ROX™, TAMRA™, TET™, Tetramethylrhodamine, or Texas Red®. A signal amplification reagent, such as tyramide (PerkinElmer), may be used to enhance the fluorescence signal. Indirect reporter molecules include biotin, which must be bound to another molecule such as streptavidin-phycoerythrin for detection. Pairs of labels, such as fluorescence resonance energy transfer pairs or dye-quencher pairs, may also be employed.

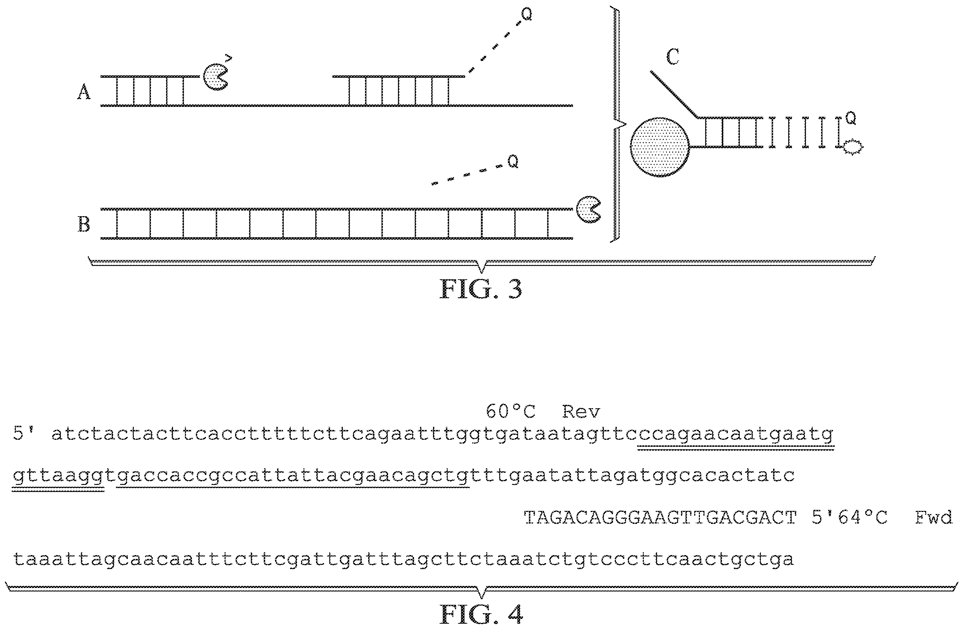

In some aspects, non-natural bases that differ from the naturally occurring bases (A, T, C, G, and U) in their hydrogen bonding pattern may be incorporated into the primers and probes described herein. One example are the isoC and isoG bases that hydrogen bond with each other, but not with natural bases. The incorporation of these non-natural bases in primers and/or probes is useful in reducing non-specific hybridization (see, *e*.*g*., FIG. 3). Methods of using such non-natural bases to assay target nucleic acids are disclosed in U.S. Patent No. 6,977,161. In one aspect, at least one of the two target-specific primers used to amplify the target nucleic acid includes at least 1, 2, 3, or 4 non-natural bases, and the complementary non-natural base is included in the amplification reaction, such that the non-natural base(s) is included in the amplification product. In such an aspect, a complementary non-natural base(s) is incorporated in the probe. The presence of complementary non-natural bases, such as isoC and isoG, in the probe and the target sequence will permit hybridization between these sequences but decrease non-specific hybridization with other sequences.

In certain aspects of the embodiments, a solid support is used. A variety of solid supports for the immobilization of biomolecules are known. For example, the solid support may be nitrocellulose, nylon membrane, glass, activated quartz, activated glass, polyvinylidene difluoride (PVDF) membrane, polystyrene substrates, polyacrylamide-based substrate, other polymers, copolymers, or crosslinked polymers such as poly(vinyl chloride), poly(methyl methacrylate), poly(dimethyl siloxane), photopolymers (which contain photoreactive species such as nitrenes, carbenes and ketyl radicals capable of forming covalent links with target molecules). A solid support may be in the form of, for example, a bead (microsphere), a column, or a chip. Molecules immobilized on planar solid supports are typically identified by their spatial position on the support. Molecules immobilized on nonplanar solid supports, such as particles or beads, are often identified by some form of encoding of the support, as discussed below. In some embodiments, a linker is placed between the target-specific probe or the anti-tag and the solid support to which it is attached.

Beads and particles may be encoded such that one subpopulation of beads or particles can be distinguished from another subpopulation. Encoding may be by a variety of techniques. For example, the beads may be fluorescently labeled with fluorescent dyes having different emission spectra and/or different signal intensities. In certain embodiments, the beads are Luminex MagPlex® microspheres or Luminex xMAP® microspheres. The size of the beads in a subpopulation may also be used to distinguish one subpopulation from another. Another method of modifying a bead is to incorporate a magnetically responsive substance, such as Fe₃O₄, into the structure. Paramagnetic and superparamagnetic microspheres have negligible magnetism in the absence of a magnetic field, but application of a magnetic field induces alignment of the magnetic domains in the microspheres, resulting in attraction of the microspheres to the field source. Combining fluorescent dyes, bead size, and/or magnetically responsive substances into the beads can further increase the number of different subpopulations of beads that can be created.

Detection of the target nucleic acid may be by a variety of techniques. In one aspect of the invention, the amplified target nucleic acids are detected using a flow cytometer. Flow cytometry is particularly well-suited where the solid support of the capture complex is a bead or other particle. In other aspects of the invention, detecting the amplified target nucleic acid comprises imaging the amplified target nucleic acid sequence bound to the capture complex in a static imaging system, such a bead array platform or a chip array platform.

The methods of the present invention may be used in multiplexed assays. In such multiplexed assays, the sample will typically comprise at least a second target nucleic acid sequence. In certain aspects of the invention, there are 1, 2, 3, 4, 5, 6, 7, 8, 9, 10, 11, 12, 13, 14, 15, 16, 17, 18, 19, 20, 21, 22, 23, 24, 25, 26, 27 ,28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 120, 140, 160, 180, 200, 220, 240, 260, 280, 300, 400, 500, 600, 700, 800, 900, 1000, or any range derivable therein, target nucleic acid sequences in the sample. As mentioned above, a target nucleic acid sequence may be any sequence of interest. One target nucleic acid sequence may be in the same gene or a different gene as another target nucleic acid sequence, and the target nucleic acid sequences may or may not overlap. Of course, a target nucleic acid sequence need not be within a gene but may be within, for example, a non-coding region of DNA. In a multiplex assay where at least a second target nucleic acid to be amplified is present in a sample, at least a second discriminating primer or primer pair is included in the reaction.

The methods of detecting the nucleic acid may comprise repeatedly extending the primer along the template nucleic acid to amplify the sequence. The amplification may be qualitative, semi-quantitative, or quantitative. In certain embodiments, the amplification may be monitored in real time (*e*.*g*., real-time PCR). The amplification cycle can be repeated until the desired amount of amplification product is produced. Typically, the amplification cycle is repeated between about 10 to 40 times. For real-time PCR, detection of the amplification products will typically be done after each amplification cycle. Although in certain aspects of the invention, detection of the amplification products may be done after only a subset of the amplification cycles, such as after every second, third, fourth, or fifth amplification cycle. Detection may also be done such that as few as 2 or more amplification cycles are analyzed or detected.

In certain embodiments, methods of quantifying an amount of nucleic acids are provided. Described herein is a method for quantifying an amount of a target nucleic acid in a sample is provided which comprises: (a) amplifying the target nucleic acid in the presence of a nucleic acid polymerase having exonuclease activity, a first target-specific primer pair comprising a first primer complementary to a first region on a first strand of the target nucleic acid and a second primer complementary to a region on a second strand of the target nucleic acid, and a target-specific probe complementary to a second region on the first strand of the target nucleic acid downstream of the first region, wherein the target-specific probe comprises a dark quencher, and further wherein the nucleic acid polymerase cleaves the first target-specific probe and releases the dark quencher from the target-specific probe when extending the first primer along the first strand of the target nucleic acid; (b) hybridizing the remaining first target-specific probe to a first reporter probe that is complementary to the first target-specific probe, said reporter probe comprising a reporter and being attached to a solid support; (c) detecting a first signal from the reporter on the solid support at a first time and a second signal from the reporter on the solid support at a second time; (d) correlating a change in signal with the amount of the target nucleic acid in the sample. In some embodiments, the method further comprises detecting at least a 3^{rd}, 4^{th}, 5^{th}, 6^{th} , 7^{th} , 8^{th} , 9^{th} , 10^{th} , 11^{th} , 12^{th}, 13^{th} , 14^{th} , 15^{th} , 16^{th} , 17^{th} , 18^{th} , 19^{th} , 20^{th} , 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, or 40^{th} signal from the reporter on the solid support at a 3^{rd}, 4^{th}, 5^{th} , 6^{th}, 7^{th} , 8^{th}, 9^{th}, 10^{th}, 11^{th}, 12^{th}, 13^{th}, 14^{th}, 15^{th}, 16^{th} , 17^{th} , 18^{th} , 19^{th} , 20^{th} , 21^{st}, 22^{nd}, 23^{rd}, 24^{th} , 25^{th}, 26^{th} , 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, or 40^{th} time. In certain aspects, the method comprises detecting a signal from the reporter on the solid support prior to extending the target-specific primers with the nucleic acid polymerase having exonuclease activity to cleave the hybridized target-specific probe and release the dark quencher from the probe. In some embodiments, the method comprises quantifying an amount of a plurality of different target nucleic acids in the sample.

Described herein is a method for quantifying an amount of a target nucleic acid in a sample is provided, which comprises: (a) amplifying the target nucleic acid in the presence of a nucleic acid polymerase having exonuclease activity, a first target-specific primer pair comprising a first primer complementary to a first region on a first strand of the target nucleic acid and a second primer complementary to a region on a second strand of the target nucleic acid, and a target-specific probe complementary to a second region on the first strand of the target nucleic acid downstream of the first region, wherein the target-specific probe comprises a dark quencher, and further wherein the nucleic acid polymerase cleaves the first target-specific probe and releases the dark quencher from the target-specific probe when extending the first primer along the first strand of the target nucleic acid; (b) hybridizing the remaining first target-specific probe to a first reporter probe that is complementary to the first target-specific probe, said reporter probe comprising a reporter and being attached to a solid support; (c) detecting a first signal from the reporter on the solid support at a first time and a second signal from the reporter on the solid support at a second time; (d) correlating a change in signal with the amount of the target nucleic acid in the sample. In some embodiments, the method further comprises detecting at least a 3^{rd}, 4^{th}, 5^{th}, 6^{th} , 7^{th} , 8^{th} , 9^{th} , 10^{th} , 11^{th} , 12^{th}, 13^{th} , 14^{th} , 15^{th}, 16^{th} , 17^{th} , 18^{th} , 19^{th} , 20^{th} , 21^{st}, 22^{nd}, 23^{rd}, 24^{th}, 25^{th}, 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, or 40^{th} signal from the reporter on the solid support at a 3^{rd}, 4^{th}, 5^{th} , 6^{th}, 7^{th}, 8^{th}, 9^{th}, 10^{th}, 11^{th}, 12^{th}, 13^{th}, 14^{th}, 15^{th}, 16^{th} , 17^{th} , 18^{th} , 19^{th} , 20^{th} , 21^{st}, 22^{nd}, 23^{rd}, 24^{th} , 25^{th} , 26^{th}, 27^{th}, 28^{th}, 29^{th}, 30^{th}, 31^{st}, 32^{nd}, 33^{rd}, 34^{th}, 35^{th}, 36^{th}, 37^{th}, 38^{th}, 39^{th}, or 40^{th} time. In certain aspects, the method comprises detecting a signal from the reporter on the solid support prior to extending the target-specific primer with the nucleic acid polymerase having exonuclease activity to cleave the hybridized target-specific probe and release the dark quencher from probe. In some embodiments, the method comprises quantifying an amount of a plurality of different target nucleic acids in the sample.

In quantitative PCR the threshold cycle (Ct) reflects the cycle number at which the fluorescence generated within a reaction crosses the threshold. It is inversely correlated to the logarithm of the initial copy number. The determination of the Ct value for each reaction is related to the baseline, background, and threshold set by the software. In some qPCR methods, a passive reference dye is used and the signal from the fluorescent reporter is divided by the signal from the reference dye to account for variability in the reaction medium. This calculation gives the normalized reporter signal (Rn). The baseline refers to the initial cycles in PCR in which there is little expected change in fluorescent signal (usually cycles 3 to 15). This baseline can be used to determine the background for each reaction. In a multiwell reaction plate, several baselines from multiple wells may be used to determine the 'baseline fluorescence' across the plate. There are many ways to use data analysis to determine when target amplification is above the background signal (crosses the threshold). Rn can be subtracted by the background signal to give ΔRn. Other supplements to data analysis that are typically employed in qPCR may be applied to the present invention. Namely, the use of endogenous and exogenous controls, housekeeping genes, standard curves, internal positive controls, no amplification controls, reverse transcription controls, nontreated controls, extraction controls, time point zeros, healthy individual controls, and negative and positive controls. These may be used in the present invention in order to perform Comparative Ct analysis ("relative quantitation") or standard curve analysis ("absolute quantitation"), the Pfaffl method, end-point quantitation, qualitative results, allelic discrimination, etc. Accounting for amplification efficiency or amplification rate may be performed by a number of methods including but not limited to: Dilution method, fluorescence increase in exponential phase, Sigmoidal or logistic curve fit, etc. The threshold may be determined by a number of methods including but not limited to the second derivative maximum method, or by a multiple of standard deviations above background, etc. Endpoint quantitative analysis could be performed by a number of methods including but not limited to: relative, absolute, competitive and comparative.

In the methods described herein, the variability in signal from well to well is not as high as in conventional bulk fluorescence measurement qPCR. In bulk fluorescent PCR, some changes in signal can be related to volume differences in each well. In certain embodiments of the present invention, volume differences will not change fluorescence attached on a solid support, and a passive bulk fluid reference dye is not needed. As multiple images are taken of spectrally identifiable particles, changes in focus and light intensity within or between imaging chambers may cause variability in signal. This can be normalized by calibration particles or passive reference particles. Calibration particles can be used to focus and optimize the light intensity or detector settings for each imaging chamber before analysis of the reaction. They can also be mixed with each reaction to normalize signal from image to image. A calibration particle is generally internally dyed with a known amount of classification dye as well as reporter dye. A passive reference particle may be used to normalize signal by subtracting or dividing from the target specific probes. A passive reference particle is generally externally dyed with probes that are designed to not hybridize or interact with any other portions of target nucleic acid in the reaction. Other particles may include those with no reporter dye, internal or external can be used to normalize for changes in bulk fluorescence which may affect the measured signal on each particle in the reaction. Sections of the imaging chamber that do not contain beads may also be used to normalize signal.

There are many ways in which the data analysis can be done. Below is an illustrative example of one method for performing data analysis for relative quantitation of mRNA. After calibration of the imaging chamber with calibration particles, one or more regions of passive reference particles and one or more regions of target specific particles as well as one or more regions specific for an endogenous control or housekeeping gene are included in an imaging chamber capable of thermal cycling. Each of the particle types is spectrally identifiable by internal classification dyes, which divide them into regions. At least 30 particles of each region are included in the reaction. The first 10 cycles of the reaction are imaged during the annealing or extension phase of the PCR cycle. A median fluorescent intensity (MFI) value is determined by taking the median of the at least 30 particles of each region. These first 10 cycles represent the baseline. The MFI of the target specific and endogenous control particles is divided by the MFI of the passive reference particle (Rn). The average Rn from the baseline is used to subtract from subsequent images as the reaction proceeds (ΔRn). A threshold is determined by taking the standard deviation (SD) of the Rn for each region and multiplying it by 10. When the ΔRn exceeds 10 SD of the baseline a Ct is recorded for each particle region. These Ct values may then be analyzed by normalizing the target specific regions to the housekeeping or endogenous control regions. This normalization is typically done by taking the difference of the Ct of the target specific region by that of the endogenous control (ΔCt). Next, if two samples are to be compared (test sample vs. control sample, or disease vs. healthy sample) then the 'delta-delta Ct' method could be used without correcting for efficiency (R = 2^{-[ΔCtsample - ΔCtcontrol]}).

Amplification efficiency may be determined either by direct or indirect methods known to those in the art and can be used to correct quantification data. Direct methods can include determining the amplification efficiency by the dilution method or by a measurement of the relative fluorescence in the exponential phase. Other indirect methods may include fitting amplification curves to a mathematical model such as sigmoidal, logistic or exponential curve fitting. In certain embodiments the quantitation of target nucleic acids is achieved using digital PCR (dPCR). In this approach the sample is partitioned so that individual nucleic acid molecules contained in the sample are localized in many separate regions, such as in individual wells in microwell plates, in the dispersed phase of an emulsion, or arrays of nucleic acid binding surfaces. Each partition will contain 0 or 1 molecule, providing a negative or positive reaction, respectively. Unlike conventional PCR, dPCR is not dependent on the number of amplification cycles to determine the initial amount of the target nucleic acid in the sample. Accordingly, dPCR eliminates the reliance on exponential data to quantify target nucleic acids and provides absolute quantification.

The present disclosure also provides compositions and kits for use in any of the disclosed methods. For example, a composition may comprise (a) at least 2, 3, 4, 5, 6, 7, 8, 9, 10, 11, 12, 13, 14, 15, 16, 17, 18, 19, 20, 21, 22, 23, 24, 25, 26, 27 ,28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 120, 140, 160, 180, 200, 220, 240, 260, 280, 300, 400, 500, 600, 700, 800, 900, 1000, or any range derivable therein, different primer-probe sets, wherein each primer-probe set comprises: (i) a first primer complementary to a first region on a first strand of a target nucleic acid, (ii) a second primer complementary to a region on a second strand of the target nucleic acid, (iii) a labeled target-specific probe, and (iv) a labeled reporter probe covalently attached to a solid support (*e*.*g*., distinguishably encoded particle), wherein the labeled target-specific probe is capable of specifically hybridizing to a second region on the first strand of the target nucleic acid, wherein the second region is downstream of the first region. The composition may further comprise a polymerase with 5' exonuclease activity. The composition may further comprise one or more negative-control (*i.e.,* passive reference) probes covalently attached to a distinguishably encoded particle. Negative-control probes are probes that are designed such that they do not specifically hybridize to any nucleic acid expected to be in a given sample. The composition may further comprise one or more positive-control probes covalently attached to a distinguishably encoded particle. Positive-control probes are probes that are designed such that they specifically hybridize to a nucleic acid expected to be in a given sample.

As described herein, a kit is provided that may comprise (a) at least 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 120, 140, 160, 180, 200, 220, 240, 260, 280, 300, 400, 500, 600, 700, 800, 900, 1000, or any range derivable therein, different primer-probe sets, wherein each primer-probe set comprises: (i) a first primer complementary to a first region on a first strand of a target nucleic acid, (ii) a second primer complementary to a region on a second strand of the target nucleic acid, (iii) a labeled target-specific probe, and (iv) a labeled reporter probe covalently attached to a solid support (*e*.*g*., a distinguishably encoded particle), wherein the labeled target-specific probe is capable of specifically hybridizing to a second region on the first strand of the target nucleic acid, wherein the second region is downstream of the first region. The kit may further comprise a polymerase with 5' exonuclease activity. The kit may further comprises one or more negative-control probes covalently attached to a distinguishably encoded particle. The kit may further comprise one or more positive-control probes covalently attached to a distinguishably encoded particle. Components of the kit may be provided in the same container or in separate containers packaged together. The kit may be an infectious disease kit, and primer-probe pairs are designed to amplify target sequences from pathogens (*e*.*g*., bacteria, viruses). The kit may be an gene expression profiling kit, and primer-probe pairs are designed to amplify target sequences from various expressed gene sequences.

As used herein the specification, "a" or "an" may mean one or more. As used herein in the claim(s), when used in conjunction with the word "comprising", the words "a" or "an" may mean one or more than one.

The use of the term "or" in the claims is used to mean "and/or" unless explicitly indicated to refer to alternatives only or the alternatives are mutually exclusive, although the disclosure supports a definition that refers to only alternatives and "and/or." As used herein "another" may mean at least a second or more.

Throughout this application, the term "about" is used to indicate that a value includes the inherent variation of error for the device, the method being employed to determine the value, or the variation that exists among the study subjects.

Other objects, features and advantages of the present invention will become apparent from the following detailed description. It should be understood, however, that the detailed description and the specific examples, while indicating preferred embodiments of the invention, are given by way of illustration only, since various changes and modifications within the spirit and scope of the invention will become apparent to those skilled in the art from this detailed description.

### BRIEF DESCRIPTION OF THE DRAWINGS

The following drawings form part of the present specification and are included to further demonstrate certain aspects of the present invention. The invention may be better understood by reference to one or more of these drawings in combination with the detailed description of specific embodiments presented herein.

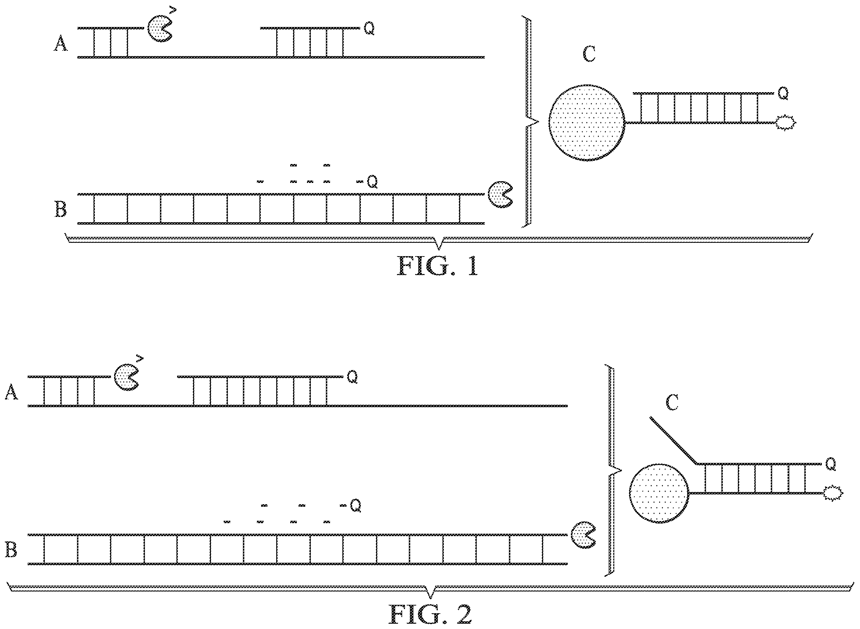

**FIG. 1** - A schematic showing an exemplary cleavage probe and reporter probe system of the embodiments. Panel A shows a target specific primer (and a bound polymerase) and target-specific probe (including a quencher) hybridized to a target nucleic acid molecule. Panel B depicts polymerization of a complimentary nucleic acid to the target nucleic acid molecule and cleavage of the target-specific probe. Panel C shows hybridization of any remaining target-specific probe to a reporter probe (which is bound to bead and includes a fluorescence moiety). In this arrangement unquenching of the fluorescence signal from the reporting probe is used to detect the presence (and quantity) of the target nucleic acid molecule.
**FIG. 2** - A schematic showing an exemplary cleavage probe and reporter probe system of the embodiments. In this example, the hybridization temperature of the target-specific probe for the target nucleic acid molecule is different than the hybridization temperature of the target-specific probe for the reporter probe. In particular, in this example the hybridization temperature of the target-specific probe for the target nucleic acid molecule is higher than the hybridization temperature of the target-specific probe for the reporter probe due to the greater number of complementary bases between the target-specific probe and the target nucleic acid molecule. Panel A shows a target specific primer (and a bound polymerase) and target-specific probe (including a quencher) hybridized to a target nucleic acid molecule. Panel B depicts polymerization of a complimentary nucleic acid to the target nucleic acid molecule and cleavage of the target-specific probe. Panel C shows hybridization of any remaining target-specific probe to a reporter probe (which is bound to bead and includes a fluorescence moiety). In this arrangement unquenching of the fluorescence signal from the reporting probe is used to detect the presence (and quantity) of the target nucleic acid molecule.
**FIG. 3** - A schematic showing an exemplary cleavage probe and reporter probe system of the embodiments. In this example, the target-specific probe and the reporter probe both include isobase positions, which are indicated by the dashed lines. Panel A shows a target specific primer (and a bound polymerase) and target-specific probe (including a quencher and isobase positions) hybridized to a target nucleic acid molecule. Panel B depicts polymerization of a complimentary nucleic acid to the target nucleic acid molecule and cleavage of a portion of the target-specific probe. The length of the isobase-containing fragment cleaved from the target-specific probe is too short to hybridize to the reporter probe at the hybridization temperature at which the uncleaved target-specific probe is hybridized to the reporter probe. Panel C shows hybridization of any remaining intact target-specific probe to a reporter probe (which is bound to bead and includes a fluorescence moiety). In this arrangement unquenching of the fluorescence signal from the reporting probe is used to detect the presence (and quantity) of the target nucleic acid molecule.
**FIG. 4** - An illustration of the forward (SEQ ID NO: 5) and reverse (SEQ ID NO: 6) primers hybridized to the amplicon (SEQ ID NO: 9) as described in Example 2. The melting temperature of the primers is also shown. The underlined sequence represents the area for probe hybridization to the strand produced in excess by the reverse primer and the double underlined sequence represents the reverse primer.

### DESCRIPTION OF ILLUSTRATIVE EMBODIMENTS

Certain aspects of the present disclosure employ hydrolysis probes for the detection of nucleic acids. Hydrolysis probes take advantage of the 5' exonuclease activity of some polymerases. During the extension or elongation phase of a PCR reaction, a polymerase, such as Taq polymerase, uses an upstream primer as a binding site and then extends. The hydrolysis probe is then cleaved during polymerase extension at its 5' end by the 5'-exonuclease activity of the polymerase (see, *e*.*g*., FIGs 1-3).

Since the fluorophore is located only on the microsphere and the hydrolysis probe comprises a quencher, there will not be an excess of fluorophore in the solution, which eliminates the need for washing steps prior to imaging. Also, since the probes are not extendable primers, they will not be susceptible to mispriming events or primer dimer formation, making them more specific than an extendable primer.

### I. Definitions

The terms "upstream" and "downstream" are used herein in relation to the synthesis of the nascent strand that is primed by a target-specific primer. Thus, for example, a target-specific probe hybridized to a region of the target nucleic acid that is "downstream" of the region of the target nucleic acid to which the primer is hybridized is located 3' of the primer and will be in the path of a polymerase extending the primer in a 5' to 3' direction.

A primer is a nucleic acid that is capable of priming the synthesis of a nascent nucleic acid in a template-dependent process. A target-specific primer refers to a primer that has been designed to prime the synthesis of a particular target nucleic acid. A primer pair refers to two primers, commonly known as a forward primer and a reverse primer or as an upstream primer and a downstream primer, which are designed to amplify a target sequence between the binding sites of the two primers on a template nucleic acid molecule. In certain embodiments, the primer has a target-specific sequence that is between 10-40, 15-30, or 18-26 nucleotides in length.

A probe is a nucleic acid that is capable of hybridizing to a complementary nucleic acid. A target-specific probe refers to a probe that has been designed to hybridize to a particular target nucleic acid. Probes present in the reaction may comprise a blocked 3' hydroxyl group to prevent extension of the probes by the polymerase. The 3' hydroxyl group may be blocked with, for example, a phosphate group, a 3' inverted dT, or a reporter. High stringency hybridization conditions may be selected that will only allow hybridization between sequences that are completely complementary.

Various aspects of the present invention use sets of complementary tag and anti-tag sequences. Which sequence in a complementary pair is called the "tag" and which is called the "anti-tag" is arbitrary. The tags and anti-tags are preferably non-cross hybridizing, *i.e*., each tag and anti-tag should hybridize only to its complementary partner, and not to other tags or anti-tags in the same reaction. Preferably, the tags and anti-tags also will not hybridize to other nucleic acids in the sample during a reaction. The tag and anti-tag sequences are also preferably designed to be isothermic, *i.e.,* of similar optimal hybridization temperature, whereby all of the tag and anti-tag sequences in a multiplex reaction will have approximately the same Tm. The proper selection of non-cross hybridizing tag and anti-tag sequences is useful in assays, particularly assays in a highly parallel hybridization environment, that require stringent non-cross hybridizing behavior. In certain embodiments, the tag and anti-tag sequences are between 6 to 60, 8 to 50, 10 to 40, 10 to 20, 12 to 24, or 20 to 30 nucleotides in length. In some embodiments, the tag and anti-tag sequences are 12, 14, 16, or 24 nucleotides in length. A number of tag and tag complement (*i.e.,* anti-tag) sequences are known in the art and may be used in the present invention. For example, U.S. Patent 7,226,737 describes a set of 210 non-cross hybridizing tags and anti-tags. In addition, U.S. Patent 7,645,868 discloses a family of 1168 tag sequences with a demonstrated ability to correctly hybridize to their complementary sequences with minimal cross hybridization. A "universal" tag or anti-tag refers to a tag or anti-tag that has the same sequence across all reactions in a multiplex reaction.

As used herein, "hybridization," "hybridizes" or "capable of hybridizing" is understood to mean the forming of a double or triple stranded molecule or a molecule with partial double or triple stranded nature. The term "anneal" as used herein is synonymous with "hybridize." As used herein "stringent conditions" or "high stringency" are those conditions that allow hybridization between or within one or more nucleic acid strands containing complementary sequences, but preclude hybridization of non-complementary sequences. Such conditions are well known to those of ordinary skill in the art, and are preferred for applications requiring high selectivity. Stringent conditions may comprise low salt and/or high temperature conditions. It is understood that the temperature and ionic strength of a desired stringency are determined in part by the length of the particular nucleic acids, the length and nucleobase content of the target sequences, the charge composition of the nucleic acids, and to the presence or concentration of formamide, tetramethylammonium chloride or other solvents in a hybridization mixture.

### II. PCR

The polymerase chain reaction (PCR) is a technique widely used in molecular biology to amplify a piece of DNA by in vitro enzymatic replication. Typically, PCR applications employ a heat-stable DNA polymerase, such as Taq polymerase. This DNA polymerase enzymatically assembles a new DNA strand from nucleotides (dNTPs) using single-stranded DNA as template and DNA primers to initiate DNA synthesis. A basic PCR reaction requires several components and reagents including: a DNA template that contains the target sequence to be amplified; one or more primers, which are complementary to the DNA regions at the 5' and 3' ends of the target sequence; a DNA polymerase (*e*.*g*., Taq polymerase) that preferably has a temperature optimum at around 70°C; deoxynucleotide triphosphates (dNTPs); a buffer solution providing a suitable chemical environment for optimum activity and stability of the DNA polymerase; divalent cations, typically magnesium ions (Mg2⁺); and monovalent cation potassium ions.

The majority of PCR methods use thermal cycling to subject the PCR sample to a defined series of temperature steps. Each cycle typically has 2 or 3 discrete temperature steps. The cycling is often preceded by a single temperature step ("initiation") at a high temperature (>90°C), and followed by one or two temperature steps at the end for final product extension ("final extension") or brief storage ("final hold"). The temperatures used and the length of time they are applied in each cycle depend on a variety of parameters. These include the enzyme used for DNA synthesis, the concentration of divalent ions and dNTPs in the reaction, and the melting temperature (Tm) of the primers. Commonly used temperatures for the various steps in PCR methods are: initialization step - 94-96°C; denaturation step - 94-98°C; annealing step - 50-65°C; extension/elongation step - 70-74°C; final elongation - 70-74°C; final hold - 4-10°C.

Real-time polymerase chain reaction, also called quantitative real time polymerase chain reaction (qPCR) or kinetic polymerase chain reaction, is used to amplify and simultaneously quantify a targeted DNA molecule. It enables both detection and quantification (as absolute number of copies or relative amount when normalized to DNA input or additional normalizing genes) of a specific sequence in a DNA sample. Real-time PCR may be combined with reverse transcription polymerase chain reaction to quantify low abundance RNAs. Relative concentrations of DNA present during the exponential phase of real-time PCR are determined by plotting fluorescence against cycle number on a logarithmic scale. Amounts of DNA may then be determined by comparing the results to a standard curve produced by real-time PCR of serial dilutions of a known amount of DNA. Various PCR and real-time PCR methods are disclosed in U.S. Patent Nos. 5,656,493; 5,994,056; 6,174,670; 5,716,784; 6,030,787; 6,174,670, and 7,955,802.

Digital PCR (dPCR) involves partitioning the sample such that individual nucleic acid molecules contained in the sample are localized in many separate regions, such as in individual wells in microwell plates, in the dispersed phase of an emulsion, or arrays of nucleic acid binding surfaces. Each partition will contain 0 or 1 molecule, providing a negative or positive reaction, respectively. Unlike conventional PCR, dPCR is not dependent on the number of amplification cycles to determine the initial amount of the target nucleic acid in the sample. Accordingly, dPCR eliminates the reliance on exponential data to quantify target nucleic acids and provides absolute quantification.

Multiplex-PCR and multiplex real-time PCR use of multiple, unique primer sets within a single PCR reaction to produce amplicons of different DNA sequences. By targeting multiple genes at once, additional information may be gained from a single test run that otherwise would require several times the reagents and more time to perform. Annealing temperatures for each of the primer sets should be optimized to work within a single reaction.

### III. Complementary tags

Some embodiments of the present invention employ complementary tag sequences (*i.e.,* tags and anti-tags) in the primers and/or probes. The proper selection of non-hybridizing tag and anti-tag sequences is useful in assays, particularly assays in a highly parallel hybridization environment, that require stringent non-cross hybridizing behavior.

Certain thermodynamic properties of forming nucleic acid hybrids are considered in the design of tag and anti-tag sequences. The temperature at which oligonucleotides form duplexes with their complementary sequences known as the Tₘ (the temperature at which 50% of the nucleic acid duplex is dissociated) varies according to a number of sequence dependent properties including the hydrogen bonding energies of the canonical pairs A-T and G-C (reflected in GC or base composition), stacking free energy and, to a lesser extent, nearest neighbor interactions. These energies vary widely among oligonucleotides that are typically used in hybridization assays. For example, hybridization of two probe sequences composed of 24 nucleotides, one with a 40% GC content and the other with a 60% GC content, with its complementary target under standard conditions theoretically may have a 10° C difference in melting temperature (Mueller *et al*., 1993). Problems in hybridization occur when the hybrids are allowed to form under hybridization conditions that include a single hybridization temperature that is not optimal for correct hybridization of all oligonucleotide sequences of a set. Mismatch hybridization of non-complementary probes can occur, forming duplexes with measurable mismatch stability (Peyret *et al*., 1999). Mismatching of duplexes in a particular set of oligonucleotides can occur under hybridization conditions where the mismatch results in a decrease in duplex stability that results in a higher Tₘ than the least stable correct duplex of that particular set. For example, if hybridization is carried out under conditions that favor the AT-rich perfect match duplex sequence, the possibility exists for hybridizing a GC-rich duplex sequence that contains a mismatched base having a melting temperature that is still above the correctly formed AT-rich duplex. Therefore, design of families of oligonucleotide sequences that can be used in multiplexed hybridization reactions must include consideration for the thermodynamic properties of oligonucleotides and duplex formation that will reduce or eliminate cross hybridization behavior within the designed oligonucleotide set.

There are a number of different approaches for selecting tag and anti-tag sequences for use in multiplexed hybridization assays. The selection of sequences that can be used as zip codes or tags in an addressable array has been described in the patent literature in an approach taken by Brenner and co-workers (U.S. Patent 5,654,413). Chetverin et al. (WO 93/17126, U.S. Patent Nos. 6,103,463 and 6,322,971) discloses sectioned, binary oligonucleotide arrays to sort and survey nucleic acids. These arrays have a constant nucleotide sequence attached to an adjacent variable nucleotide sequence, both bound to a solid support by a covalent linking moiety. Parameters used in the design of tags based on subunits are discussed in Barany et al. (WO 9731256). A multiplex sequencing method has been described in U.S. Patent 4,942,124. This method uses at least two vectors that differ from each other at a tag sequence.

U.S. Patent 7,226,737 describes a set of 210 non-cross hybridizing tags and anti-tags. U.S. Published Application No. 2005/0191625 discloses a family of 1168 tag sequences with a demonstrated ability to correctly hybridize to their complementary sequences with minimal cross hybridization. U.S. Publication No. 2009/0148849 anti-tags, and capture complexes in the amplification of nucleic acid sequences.

A population of oligonucleotide tag or anti-tag sequences may be conjugated to a population of primers or other polynucleotide sequences in several different ways including, but not limited to, direct chemical synthesis, chemical coupling, ligation, amplification, and the like. Sequence tags that have been synthesized with target specific primer sequences can be used for enzymatic extension of the primer on the target for example in PCR amplification. A population of oligonucleotide tag or anti-tag sequences may be conjugated to a solid support by, for example, surface chemistries on the surface of the support.

### IV. Solid supports

In certain embodiments, the probes and/or primers may be attached to a solid support. Such solid supports may be, for example, microspheres (*i.e.,* beads) or other particles such as microparticles, gold or other metal nanoparticles, quantum dots, or nanodots. In certain aspects, the particles may be magnetic, paramagnetic, or super paramagnetic. Examples of microspheres, beads, and particles are illustrated in U.S. Patent Nos. 5,736,330 to Fulton, 5,981,180 to Chandler et al., 6,057,107 to Fulton, 6,268,222 to Chandler et al., 6,449,562 to Chandler et al., 6,514,295 to Chandler et al., 6,524,793 to Chandler et al., and 6,528,165 to Chandler.

The particles may be encoded with a label. In certain embodiments, the present invention is used in conjunction with Luminex® xMAP® and MagPlex™ technologies. The Luminex xMAP technology allows the detection of nucleic acid products immobilized on fluorescently encoded microspheres. By dyeing microspheres with 10 different intensities of each of two spectrally distinct fluorochromes, 100 fluorescently distinct populations of microspheres are produced. These individual populations (sets) can represent individual detection sequences and the magnitude of hybridization on each set can be detected individually. The magnitude of the hybridization reaction is measured using a third reporter, which is typically a third spectrally distinct fluorophore. In embodiments in which a labeled hydrolysis probe is attached to the microsphere, hybridization and hydrolysis of the probe results in a decrease in signal from the third reporter. As both the microspheres and the reporter molecules are labeled, digital signal processing allows the translation of signals into real-time, quantitative data for each reaction. The Luminex technology is described, for example, in U.S. Patents 5,736,330, 5,981,180, and 6,057,107. Luminex® MagPlex™ microspheres are superparamagnetic microspheres that are fluorescently encoded using the xMAP® technology discussed above. The microspheres contain surface carboxyl groups for covalent attachment of ligands (or biomolecules).

Alternatively, the solid support may be a planar array such as a gene chip or microarray (*see*, *e*.*g*., Pease *et al*., 1994; Fodor *et al*., 1991). The identity of nucleic acids on a planar array is typically determined by it spatial location on the array. Microsphere based assays may also be analyzed on bead array platforms. In general, bead array platforms image beads and analytes distributed on a substantially planar array. In this way, imaging of bead arrays is similar to the gene chips discussed above. However, in contrast to gene chips where the analyte is typically identified by its spatial position on the array, bead arrays typically identify the analyte by the encoded microsphere to which it is bound.

The ability to directly synthesize on or attach polynucleotide probes to solid substrates is well known in the art. See U.S. Pat. Nos. 5,837,832 and 5,837,860. A variety of methods have been utilized to either permanently or removably attach the probes to the substrate. Exemplary methods include: the immobilization of biotinylated nucleic acid molecules to avidin/streptavidin coated supports (Holmstrom, 1993), the direct covalent attachment of short, 5'-phosphorylated primers to chemically modified polystyrene plates (Rasmussen *et al*., 1991), or the precoating of the polystyrene or glass solid phases with poly-L-Lys or poly L-Lys, Phe, followed by the covalent attachment of either amino- or sulfhydryl-modified oligonucleotides using bi-functional crosslinking reagents (Running *et al*., 1990; Newton *et al*., 1993). Numerous materials may be used as solid supports, including reinforced nitrocellulose membrane, activated quartz, activated glass, polyvinylidene difluoride (PVDF) membrane, polystyrene substrates, polyacrylamide-based substrate, other polymers such as poly(vinyl chloride), poly(methyl methacrylate), poly(dimethyl siloxane), photopolymers (which contain photoreactive species such as nitrenes, carbenes and ketyl radicals capable of forming covalent links with target molecules.

### V. Detection

Various aspects of the present invention relate to the direct or indirect detection of one or more target nucleic acids by detecting an increase or decrease in a signal. The detection techniques employed will depend on the type of reporter and platform (*e*.*g*., spectrally encoded beads, microarray, etc.). Flow cytometry, for example, is particularly useful in the analysis of microsphere based assays. Flow cytometry involves the separation of cells or other particles, such as microspheres, in a liquid sample. Generally, the purpose of flow cytometry is to analyze the separated particles for one or more characteristics. The basic steps of flow cytometry involve the direction of a fluid sample through an apparatus such that a liquid stream passes through a sensing region. The particles should pass one at a time by the sensor and are categorized based on size, refraction, light scattering, opacity, roughness, shape, fluorescence, etc.

In the context of the Luminex xMAP® system, flow cytometry can be used for simultaneous sequence identification and hybridization quantification. Internal dyes in the microspheres are detected by flow cytometry and used to identify the specific nucleic acid sequence to which a microsphere is coupled. The label on the target nucleic acid molecule or probe is also detected by flow cytometry and used to determine hybridization to the microsphere.

Methods of flow cytometry are well known in the art and are described, for example, in U.S. patents. U.S. Pat. Nos. 5,981,180, 4,284,412; 4,989,977; 4,498,766; 5,478,722; 4,857,451; 4,774,189; 4,767,206; 4,714,682; 5,160,974; and 4,661,913. The measurements described herein may include image processing for analyzing one or more images of particles to determine one or more characteristics of the particles such as numerical values representing the magnitude of fluorescence emission of the particles at multiple detection wavelengths. Subsequent processing of the one or more characteristics of the particles such as using one or more of the numerical values to determine a token ID representing the multiplex subset to which the particles belong and/or a reporter value representing a presence and/or a quantity of analyte bound to the surface of the particles can be performed according to the methods described in U.S. Patent Nos. 5,736,330 to Fulton, 5,981,180 to Chandler et al., 6,449,562 to Chandler et al., 6,524,793 to Chandler et al., 6,592,822 to Chandler, and 6,939,720 to Chandler et al..

In one example, techniques described in U.S. Patent No. 5,981,180 to Chandler et al. may be used with the fluorescent measurements described herein in a multiplexing scheme in which the particles are classified into subsets for analysis of multiple analytes in a single sample. Additional examples of systems that may be configured as described herein (*e.g.,* by inclusion of an embodiment of an illumination subsystem described herein) are illustrated in U.S. Patents Nos. 5,981,180 to Chandler et al., 6,046,807 to Chandler, 6,139,800 to Chandler, 6,366,354 to Chandler, 6,411,904 to Chandler, 6,449,562 to Chandler et al., and 6,524,793 to Chandler et al..

Microspheres may also be analyzed on array platforms that image beads and analytes distributed on a substantially planar array. In this way, imaging of bead arrays is similar to imaging of gene chips. However, in contrast to gene chips where the analyte is identified by its spatial position (*i.e.,* x, y coordinate) on the array, bead arrays typically identify the analyte by the encoded microsphere to which it is bound. Examples of commercially available bead array systems include Luminex's MAGPIX®, and Illumina's BeadXpress™ Reader and BeadStation 500™. Once beads are in a planar layer, they can be identified by their "coding" (either in the form of embedded dyes, or other methods that create unique signals for each bead type). Following or preceding the resolution of the "code" of the bead, the signal can be measured and these two measurements coupled to determine the hybridization of a particular nucleic acid to the bead.

### VI. Kits

The present disclosure also provides kits containing components for use with the amplification and detection methods disclosed herein. Any of the components disclosed here in may be combined in a kit. In certain embodiments the kits comprise a plurality of primers for priming amplification of a plurality of nucleic acid targets, and a plurality of probes complementary to the plurality of nucleic acid targets. In some embodiments, the probes are immobilized on a solid support(s). In one embodiment, a plurality of probes are attached to a plurality of encoded magnetic beads such that the identity of each probe is known from the encoded magnetic bead on which it is immobilized. In certain embodiments, the kit also comprises a labeling agent. In certain embodiments the kits comprise probes that are not attached to a solid support. In some embodiments the kit comprises an imaging chamber, which may be a disposable imaging chamber, for use in an imaging system.

The kits will generally include at least one vial, test tube, flask, bottle, syringe or other container, into which a component may be placed, and preferably, suitably aliquoted. Where there is more than one component in the kit, the kit also will generally contain a second, third or other additional containers into which the additional components may be separately placed. However, various combinations of components may be comprised in a container. The kits of the present disclosure also will typically include packaging for containing the various containers in close confinement for commercial sale. Such packaging may include cardboard or injection or blow molded plastic packaging into which the desired containers are retained.

A kit may also include instructions for employing the kit components. Instructions may include variations that can be implemented.

### VII. Examples

### Example 1 -

As shown in FIGs. 1-3, a probe that is complementary to a target region within an amplicon having a quencher attached thereto is used as a hydrolysis probe during PCR amplification. This same probe is also complimentary to probe on a microsphere containing a fluorophore at one end. In the event that an amplicon is generated, the quenching probe in solution will hybridize to the amplicon and undergo subsequent hydrolysis by the exonuclease activity of the polymerase. In a post-PCR hybridization event to the microspheres, the microspheres whose complimentary quenching probes have been cleaved will have an increase in fluorescence by virtue of the absence of the complimentary quencher probe.

In this assay chemistry, the fluorophore is located only on the microspheres so that there is no fluorophore to image through in solution. Second, the probes are hydrolysis probes and are not extendable.

In order for the assay to detect a depleting probe in solution, the amount of probe to start with must be under the saturation of hybridization to the microspheres. Determining this saturation limit will be performed with a quencher-fluorophore combination in PCR solution.

Next, whether the saturation limit is effective for hybridization to the amplicon will be tested. However, the hydrolysis of the probes in cumulative over the course of the reaction, which may counteract any concentration dynamic range limitations.

Luminex MagPlex® Microspheres are coupled to amine-modified oligonucleotide probes according to the manufacturer's instructions.

Microsphere region 25 is coupled to a probe specific for *Staphylococcus epidermidis*: 5'- /5AmMC12/ GTA ATA ATG GCG GTG GTC/3Cy3Sp/-3' (SEQ ID NO: 1)

Microsphere region 54 is coupled to a probe that was designed to not hybridize to Staphylococcus epidermidis: 5'-/5AmMC12/GAT TGT AAG ATT TGA TAA AGT GTA/3Cy3Sp/-3' (SEQ ID NO: 2)

A solution phase probe includes a probe that is partly complementary to the probe on microsphere region 25 and fully complementary to the *Staphylococcus epidermidis* target amplicon: 5'/BHQ2/GAC CAC CGC CAT TAT TAC GAA CAG CTG-3' (SEQ ID NO: 3)

An additional solution phase probe includes a probe that is complementary to the probe on region 54: 5'/BHQ2/TAC ACT TTA TCA AAT CTT ACA ATC-3' (SEQ ID NO: 4)

Next a PCR Master mix is made for each reaction including:

| | |
|---|---|
| 2x TaqMan® Master Mix (Applied Biosystems) | 12.5 µL |
| Water | 5.7 µL |
| 50 mM MgCl₂ | 2.0 µL |
| 20x Primer Mix | 1.3 µL |
| 2500 beads/µL per region | 1.0 µL |

The 20x Primer Mix contains the following ratios per µL:

| | |
|---|---|
| TE pH8.0 | 0.64 µL |
| 100 µM Forward Primer | 0.18 µL |
| 100 µM Reverse Primer | 0.18 µL |

The Forward Primer has the following oligonucleotide sequence: 5'-TCA GCA GTT GAA GGG ACA GAT-3' (SEQ ID NO: 5)

The Reverse Primer has the following oligonucleotide sequence: 5'-CCA GAA CAA TGA ATG GTT AAG G-3' (SEQ ID NO: 6)

The template can be purchased from ATCC # 12228D-5 (S. *epidermidis* purified DNA). 2.5 µL of template in water are added to each "template" PCR reaction (2 ng per reaction), and 2.5 µL water alone are added to the "no template" PCR reactions.

The following thermal cycling protocol is used on an ABI Step One Plus ThermalCycler:
50°C for 2 min.
95°C for 10 min.
Followed by 35 cycles of a two step PCR
95°C for 15 sec.
60°C for 1 min.

After PCR, the reaction mix is taken directly to a Luminex instrument, allowed to hybridize for 10 minutes at room temperature and analyzed for Median Fluorescent Intensity (MFI) values using 100 microspheres per MFI data point. The delta MFI between the control microsphere (region 54) and the target specific microsphere (region 25) is used to determine positivity or negativity of the reaction based on predetermined cutoff thresholds.

### Example 2 - Dual-Phase Chemistry studies

Studies were performed to assess dual-phase PCR chemistry. A PCR reaction including beads coupled with Cy3 labeled fluorescent probes was used to assess whether complimentary probes labeled with a BHQ2 quencher would be consumed in the reaction by hybridization or hydrolysis to the target amplicon generated. In the case where template is present, the signal on the particle should increase because the complementary quenching probe is consumed.

The following beads with their respective probe sets were used in the PCR reaction:
Bead 33: 5'-/5Cy3/GAC CAC CGC CAT TAT TAC G/3AmMC6T/-3' (SEQ ID NO: 7)
Bead 45: 5'-/5Cy3/GAT TGT AAG ATT TGA TAA AGT GTA /3AmMO/-3' (SEQ ID NO: 2)
Bead 33 is partially complimentary to a probe that is specific for the *S. epidermidis* amplicon:
5'- CAG CTG TTC GTA ATA ATG GCG GTG GTC /3BHQ_2/ -3' (SEQ ID NO: 8)
There was no complimentary quenching oligonucleotide to hybridize to the probe on bead 45.

Results were obtained by reading the results of the PCR reaction on a MAGPIX® instrument after 15 minutes of hybridization at 40°C. Two conditions were tested: with beads in PCR and leaving the beads out of the PCR reaction, but adding them directly after the cycling was complete. As shown below in Table 1, the signal on the non-quenched, non-specific, bead 45 was diminished when beads were present during PCR as compared to when the beads were added after PCR, indicating that there was some general degradation or quenching as a result of the PCR process. Table 1 also shows that the no-template reactions for bead 33 in the beads after PCR scenario was lower than in the beads in PCR scenario, indicating that something was partially inhibiting the hybridization of the quencher to bead 33 in the beads in PCR scenario. The quencher was intact, however, because it hybridized as expected in the beads after PCR scenario. Despite these issues, the assay was able to detect the presence of 40k copies of *S. epidermidis* by comparing the template to no template signals. This level of sensitivity was achievable both when PCR was performed in the presence of beads or when the beads were added after amplification.

**Table 1. PCR Results.**

| **Beads in PCR** | **Bead 33- Quenched and specific (MFI)** | **Bead 45 - no quencher and non-specific (MFI)** |
|---|---|---|
| template | 3650 | 4017 |
| no template | 3173 | 3972.5 |
| template | 3491 | 3966 |
| no template | 3059 | 3966 |
| template | 3507 | 3948 |
| no template | 3078 | 3935.5 |

| **Beads put in after PCR** | | |
|---|---|---|
| template | 2486 | 5690 |
| no template | 1454.5 | 5721 |
| template | 2551 | 5655 |
| no template | 1450 | 5682 |
| template | 2593 | 5616 |
| no template | 1421 | 5630 |

The forward and reverse primers hybridized to the amplicon and had melting temperatures (Tₘs) as shown in FIG. 4. The reverse primer (5'-CCAGAACAATGAATGGTTAAGG-3' (SEQ ID NO: 6)) was in excess (400nM) while the forward primer 5'-TCAGCAGTTGAAGGGACAGAT-3' (SEQ ID NO: 5)) was at a lower concentration (50nM). As indicated in FIG. 4, the underlined sequence represents the area for probe hybridization to the strand produced in excess by the reverse primer and the double-underlined sequence represents the reverse primer. 1x PCR buffer contained: 10 mM Tris-HCl, 50 mM KCl, 1.5 mM MgCl₂, pH 8.3 @ 25°C.

The PCR mix formula for each reaction was:

| | Stock | Final | Vol (µL) |
|---|---|---|---|
| PCR buffer | 10x | 1x | 4.0 |
| dNTP | 10mM | 300µM | 1.2 |
| MgCl2 | 40mM | 2mM | 2.0 |
| Hot start taq (NEB M0495L) | | | 0.25 |
| primer mix | 20x | 400/50nM | 2.0 |
| beads | 2500/ µL | | 1.0 |
| probe | 20x | 600fmol/rxn | 2.0 |
| Water | | | 17.55 |
| template | | | 10 |
| | | Total volume: | 40 |

The following thermal cycling protocol was used on a thermalcycling instrument for 40 cycles in slow mode:
95°C for 3 min.
95°C for 15 sec.
60°C for 45 sec.

### REFERENCES

U.S. Patent Nos. 4,942,124; 4,284,412; 4,989,977; 4,498,766; 5,478,722; 4,857,451; 4,774,189; 4,767,206; 4,714,682; 5,160,974; 4,661,913; 5,654,413; 5,656,493; 5,716,784; 5,736,330; 5,837,832; 5,837,860; 5,981,180; 5,994,056; 5,736,330; 5,981,180; 6,057,107; 6,030,787; 6,046,807; 6,057,107; 6,103,463; 6,139,800; 6,174,670; 6,268,222; 6,322,971; 6,366,354; 6,411,904; 6,449,562; 6,514,295; 6,524,793; 6,528,165; 6,592,822; 6,939,720; 6,977,161; 7,226,737; 7,645,868; and 7,955,802
U.S. Published Application Nos. 2005/0191625; and 2009/0148849
Fodor et al., "Light-directed, spatially addressable parallel chemical synthesis," Science, 251(4995):767-73, 1991.
Holmstrom et al., "A highly sensitive and fast nonradioactive method for detection of polymerase chain reaction products," Anal. Biochem., 209(2):278-83, 1993.
Running et al., "A procedure for productive coupling of synthetic oligonucleotides to polystyrene microtiter wells for hybridization capture," Biotechniques, 8(3):276, 279, 1990.
Pease et al., "Light-generated oligonucleotide arrays for rapid DNA sequence analysis," Proc. Natl. Acad. Sci. USA, 91(11):5022-6, 1994.
Peyret et al., "Nearest-neighbor thermodynamics and NMR of DNA sequences with internal A.A, C.C, G.G, and T.T mismatches," Biochemstry, 38(12):3468-77, 1999.
International (PCT) Publication Nos. WO 93/17126; and WO 97/31256

## Claims

1. A method for detecting a target nucleic acid in a sample comprising:
(a) contacting the sample with a first target-specific primer complementary to a first region on a first strand of the target nucleic acid, a target-specific probe complementary to a second region on the first strand of the target nucleic acid downstream of the first region, and a second target-specific primer complementary to a region on a second strand of the target nucleic acid under conditions suitable for hybridization of the target nucleic acid with the first target-specific primer, the target-specific probe and the second target-specific primer, wherein the target-specific probe comprises a quencher and wherein the 3' end of the target-specific probe comprises a modification to prevent polymerase extension of the probe;
(b) performing a polymerase chain reaction (PCR) with a nucleic acid polymerase having exonuclease activity;
(c) cleaving the hybridized target-specific probe by extension of the target-specific primer with said nucleic acid polymerase to release the quencher from the target-specific probe;
(d) hybridizing any remaining target-specific probe to a reporter probe that is complementary to the target-specific probe, said reporter probe comprising a reporter and being attached to a solid support, wherein the reporter is a fluorophore; and
(e) detecting the target nucleic acid by detecting a change in signal from the reporter in association with the solid support;
wherein the hybridization temperature of the target-specific probe for the target nucleic acid molecule is higher than the hybridization temperature of the target-specific probe for the reporter probe.

2. The method of claim 1, wherein the reporter probe and the target-specific probe comprise both natural bases and isobases.

3. The method of claim 1, wherein the solid support is an encoded bead.

4. The method of claim 1, wherein the change in the signal is an increase in a fluorescent signal.

5. The method of claim 1, further comprising detecting a reference signal from a distinct reporter on a non-hybridizing probe attached to a solid support.

6. The method of claim 5, wherein the non-hybridizing probe is attached to a spatially discrete location on the same solid support to which the reporter probe is attached.

7. The method of claim 5, wherein the non-hybridizing probe is attached to a different solid support than that to which the reporter probe is attached.

8. The method of claim 7, wherein the different solid supports are different encoded beads.

9. The method of claim 1, wherein the target nucleic acid is a first target nucleic acid, the quencher is a first quencher, the reporter probe is a first reporter probe, the reporter is a first reporter, the solid support is a first solid support, and the method further comprises:
(a) contacting the sample with a third target-specific primer complementary to a first region on a first strand of a second target nucleic acid, a second target-specific probe complementary to a second region on the first strand of the second target nucleic acid downstream of the first region, and a fourth target-specific primer complementary to a region on a second strand of the second target nucleic acid under conditions suitable for hybridization of the second target nucleic acid with the third target-specific primer, the second target-specific probe, and the fourth target-specific primer wherein the second target-specific probe comprises a second quencher;
(b) performing a polymerase chain reaction (PCR) with a nucleic acid polymerase having exonuclease activity;
(c) cleaving the second hybridized target-specific probe with said nucleic acid polymerase to release the second quencher from the second target-specific probe;
(d) hybridizing any remaining second target-specific probe to a second reporter probe that is complementary to the second target-specific probe, said second reporter probe comprising a second reporter and being attached to a second solid support, wherein the second reporter is a fluorophore; and
(e) detecting the second target nucleic acid by detecting a chance in signal from the second reporter associated with the second solid support.

10. The method of claim 9, wherein the first solid support and the second solid support are spatially discrete locations on one solid support.

11. The method of claim 9, wherein the first solid support is physically separate from the second solid support.

12. The method of claim 9, wherein the first reporter and the second reporter are the same.

## Patentansprüche

1. Verfahren zum Nachweisen einer Zielnukleinsäure in einer Probe, umfassend:
(a) Inkontaktbringen der Probe mit einem ersten zielspezifischen Primer, der zu einer ersten Region auf einem ersten Strang der Zielnukleinsäure komplementär ist, einer zielspezifischen Sonde, die zu einer zweiten Region auf dem ersten Strang der Zielnukleinsäure stromabwärts von der ersten Region komplementär ist, und einem zweiten zielspezifischen Primer, der zu einer Region auf einem zweiten Strang der Zielnukleinsäure komplementär ist, unter Bedingungen, die für eine Hybridisierung der Zielnukleinsäure mit dem ersten zielspezifischen Primer, der zielspezifischen Sonde und dem zweiten zielspezifischen Primer geeignet sind, wobei die zielspezifische Sonde einen Quencher umfasst und wobei das 3'-Ende der zielspezifischen Sonde eine Modifikation umfasst, so dass eine Polymeraseverlängerung der Sonde verhindert wird;
(b) Durchführen einer Polymerasekettenreaktion (PCR) mit einer Nukleinsäure-Polymerase mit Exonuklease-Aktivität;
(c) Spalten der hybridisierten zielspezifischen Sonde durch Verlängerung des zielspezifischen Primers mit der Nukleinsäure-Polymerase, so dass der Quencher aus der zielspezifischen Sonde freigesetzt wird;
(d) Hybridisieren noch verbliebener zielspezifischer Sonde an eine Reportersonde, die zur zielspezifischen Sonde komplementär ist, wobei die Reportersonde einen Reporter umfasst und an einen festen Träger gebunden ist, wobei es sich bei dem Reporter um einen Fluorophor handelt; und
(e) Nachweisen der Zielnukleinsäure durch Nachweisen einer Änderung des Signals vom Reporter in Assoziation mit dem festen Träger;
wobei die Hybridisierungstemperatur der zielspezifischen Sonde für das Zielnukleinsäuremolekül höher als die Hybridisierungstemperatur der zielspezifischen Sonde für die Reportersonde ist.

2. Verfahren nach Anspruch 1, wobei die Reportersonde und die zielspezifische Sonde sowohl natürliche Basen als auch Isobasen umfassen.

3. Verfahren nach Anspruch 1, wobei es sich bei dem festen Träger um ein codiertes Kügelchen handelt.

4. Verfahren nach Anspruch 1, wobei es sich bei der Änderung des Signals um einen Anstieg eines Fluoreszenzsignals handelt.

5. Verfahren nach Anspruch 1, ferner umfassend Nachweisen eines Referenzsignals von einem speziellen Reporter auf einer nicht hybridisierenden Sonde, die an einen festen Träger gebunden ist.

6. Verfahren nach Anspruch 5, wobei die nicht hybridisierende Sonde an einen räumlich getrennten Ort auf dem gleichen festen Träger, an den die Reportersonde gebunden ist, gebunden ist.

7. Verfahren nach Anspruch 5, wobei die nicht hybridisierende Sonde an einen anderen festen Träger als den, an den die Reportersonde gebunden ist, gebunden ist.

8. Verfahren nach Anspruch 7, wobei es sich bei den unterschiedlichen Trägern um unterschiedliche codierte Kügelchen handelt.

9. Verfahren nach Anspruch 1, wobei es sich bei der Zielnukleinsäure um eine erste Zielnukleinsäure, beim Quencher um einen ersten Quencher, bei der Reportersonde um eine erste Reportersonde, beim Reporter um einen ersten Reporter, beim festen Träger um einen ersten festen Träger handelt und das Verfahren ferner Folgendes umfasst:
(a) Inkontaktbringen der Probe mit einem dritten zielspezifischen Primer, der zu einer ersten Region auf einem ersten Strang einer zweiten Zielnukleinsäure komplementär ist, einer zweiten zielspezifischen Sonde, die zu einer zweiten Region auf dem ersten Strang der zweiten Zielnukleinsäure stromabwärts von der ersten Region komplementär ist, und einem vierten zielspezifischen Primer, der zu einer Region auf einem zweiten Strang der zweiten Zielnukleinsäure komplementär ist, unter Bedingungen, die für eine Hybridisierung der zweiten Zielnukleinsäure mit dem dritten zielspezifischen Primer, der zweiten zielspezifischen Sonde und dem vierten zielspezifischen Primer geeignet sind, wobei die zweite zielspezifische Sonde einen zweiten Quencher umfasst;
(b) Durchführen einer Polymerasekettenreaktion (PCR) mit einer Nukleinsäure-Polymerase mit Exonuklease-Aktivität;
(c) Spalten der zweiten hybridisierten zielspezifischen Sonde durch Verlängerung des zielspezifischen Primers mit der Nukleinsäure-Polymerase, so dass der zweite Quencher aus der zweiten zielspezifischen Sonde freigesetzt wird;
(d) Hybridisieren noch verbliebener zweiter zielspezifischer Sonde an eine zweite Reportersonde, die zur zweiten zielspezifischen Sonde komplementär ist, wobei die zweite Reportersonde einen zweiten Reporter umfasst und an einen zweiten festen Träger gebunden ist, wobei es sich bei dem zweiten Reporter um einen Fluorophor handelt; und
(e) Nachweisen der zweiten Zielnukleinsäure durch Nachweisen einer Änderung des Signals vom zweiten Reporter, der mit dem zweiten festen Träger assoziiert ist.

10. Verfahren nach Anspruch 9, wobei es sich bei dem ersten festen Träger und dem zweiten festen Träger um räumlich getrennte Orte auf einem einzelnen festen Träger handelt.

11. Verfahren nach Anspruch 9, wobei der erste feste Träger vom zweiten festen Träger physikalisch getrennt ist.

12. Verfahren nach Anspruch 9, wobei der erste Reporter und der zweite Reporter identisch sind.

## Revendications

1. Procédé de détection d'un acide nucléique cible dans un échantillon comprenant :
(a) la mise en contact de l'échantillon avec une première amorce spécifique à une cible complémentaire d'une première région sur un premier brin de l'acide nucléique cible, une sonde spécifique à une cible complémentaire d'une deuxième région sur le premier brin de l'acide nucléique cible en aval de la première région, et une deuxième amorce spécifique à une cible complémentaire d'une région sur un deuxième brin de l'acide nucléique cible dans des conditions adaptées pour l'hybridation de l'acide nucléique cible avec la première amorce spécifique à une cible, la sonde spécifique à une cible et la deuxième amorce spécifique à une cible, dans lequel la sonde spécifique à une cible comprend un inactiveur et dans lequel l'extrémité 3' de la sonde spécifique à une cible comprend une modification pour empêcher l'extension par polymérase de la sonde ;
(b) la conduite d'une réaction de polymérase en chaîne (PCR) avec une acide nucléique polymérase ayant une activité exonucléase ;
(c) le clivage de la sonde spécifique à une cible hybridée par extension de l'amorce spécifique à une cible avec ladite acide nucléique polymérase pour libérer l'inactiveur de la sonde spécifique à une cible ;
(d) l'hybridation de la sonde spécifique à une cible résiduelle éventuelle à une sonde de rapporteur qui est complémentaire de la sonde spécifique à une cible, ladite sonde de rapporteur comprenant un rapporteur et étant liée à un support solide, dans lequel le rapporteur est un fluorophore ; et
(e) la détection de l'acide nucléique cible par détection d'un changement de signal provenant du rapporteur associé au support solide ;
dans lequel la température d'hybridation de la sonde spécifique à une cible pour la molécule d'acide nucléique est supérieure à la température d'hybridation de la sonde spécifique à une cible pour la sonde de rapporteur.

2. Procédé selon la revendication 1, dans lequel la sonde de rapporteur et la sonde spécifique à une cible comprennent à la fois des bases naturelles et des isobases.

3. Procédé selon la revendication 1, dans lequel le support solide est une bille codée.

4. Procédé selon la revendication 1, dans lequel le changement du signal est une augmentation d'un signal fluorescent.

5. Procédé selon la revendication 1, comprenant en outre la détection d'un signal de référence provenant d'un rapporteur distinct sur une sonde non hybridante liée à un support solide.

6. Procédé selon la revendication 5, dans lequel la sonde non hybridante est liée à un emplacement spatialement discret sur le même support solide auquel la sonde de rapporteur est liée.

7. Procédé selon la revendication 5, dans lequel la sonde non hybridante est liée à un support solide différent de celui auquel la sonde de rapporteur est liée.

8. Procédé selon la revendication 7, dans lequel les différents supports solides sont différentes billes codées.

9. Procédé selon la revendication 1, dans lequel l'acide nucléique cible est un premier acide nucléique cible, l'inactiveur est un premier inactiveur, la sonde de rapporteur est une première sonde de rapporteur, le rapporteur est un premier rapporteur, le support solide est un premier support solide, et le procédé comprend en outre :
(a) la mise en contact de l'échantillon avec une troisième amorce spécifique à une cible complémentaire d'une première région sur un premier brin d'un deuxième acide nucléique cible, une deuxième sonde spécifique à une cible complémentaire d'une deuxième région sur le premier brin du deuxième acide nucléique cible en aval de la première région, et une quatrième amorce spécifique à une cible complémentaire d'une région sur un deuxième brin du deuxième acide nucléique cible dans des conditions adaptées pour l'hybridation du deuxième acide nucléique cible avec la troisième amorce spécifique à une cible, la deuxième sonde spécifique à une cible, et la quatrième amorce spécifique à une cible dans lequel la deuxième sonde spécifique à une cible comprend un deuxième inactiveur ;
(b) la conduite d'une réaction de polymérase en chaîne (PCR) avec une acide nucléique polymérase ayant une activité exonucléase ;
(c) le clivage de la deuxième sonde spécifique à une cible hybridée avec ladite acide nucléique polymérase pour libérer le deuxième inactiveur de la deuxième sonde spécifique à une cible ;
(d) l'hybridation de la deuxième sonde spécifique à une cible résiduelle éventuelle à une deuxième sonde de rapporteur qui est complémentaire de la deuxième sonde spécifique à une cible, ladite deuxième sonde de rapporteur comprenant un deuxième rapporteur et étant liée à un deuxième support solide, le deuxième rapporteur étant un fluorophore ; et
(e) la détection du deuxième acide nucléique cible par détection d'un changement de signal du deuxième rapporteur associé au deuxième support solide.

10. Procédé selon la revendication 9, dans lequel le premier support solide et le deuxième support solide sont des emplacements spatialement discrets sur un support solide.

11. Procédé selon la revendication 9, dans lequel le premier support solide est physiquement séparé du deuxième support solide.

12. Procédé selon la revendication 9, dans lequel le premier rapporteur et le deuxième rapporteur sont identiques.
